# EUROPEAN PATENT SPECIFICATION

(11) **EP 3 358 006 B1**
(45) Date of publication and mention of the grant of the patent: **31.03.2021**
(21) Application number: 17154809.2
(22) Date of filing: 06.02.2017
(51) Int. Cl.: C12N 5/0775

(54) **METHOD AND MEDIUM FOR CULTIVATING PRIMARY CELLS IN VITRO**
VERFAHREN UND MEDIUM ZUR IN-VITRO-KULTIVIERUNG VON PRIMÄREN ZELLEN
PROCÉDÉ ET MILIEU DE CULTURE DE CELLULES PRIMAIRES IN VITRO

(43) Date of publication of application: 08.08.2018
(73) Proprietor: Mares Ltd., 48268 Greven (DE)
(72) Inventor: BOARD, Mary, Warwickshire CV376PB (GB); VAN DEN BOS, Christian, 48268 Greven (DE)
(74) Representative: Remus, Alvaro Johannes

(56) References cited:
- Raven Lopes-Cardozo Matthijs ET AL: "Acetoacetate and Glucose as Lipid Precursors and Energy Substrates in Primary Cultures of Astrocytes and Neurons from Mouse Cerebral Cortex", , 1 January 1986 (1986-01-01), XP055376948, Retrieved from the Internet: URL:https://www.ncbi.nlm.nih.gov/pubmed/30 81684 [retrieved on 2017-05-30]
- GABRIELE D MAURER ET AL: "Differential utilization of ketone bodies by neurons and glioma cell lines: a rationale for ketogenic diet as experimental glioma therapy", BMC CANCER, BIOMED CENTRAL, LONDON, GB, vol. 11, no. 1, 26 July 2011 (2011-07-26), page 315, XP021105105, ISSN: 1471-2407, DOI: 10.1186/1471-2407-11-315
- FINE EUGENE J ET AL: "Acetoacetate reduces growth and ATP concentration in cancer cell lines which over-express uncoupling protein 2", CANCER CELL INTERNATIONAL, BIOMED CENTRAL, LONDON, GB, vol. 9, no. 1, 29 May 2009 (2009-05-29), page 14, XP021058488, ISSN: 1475-2867, DOI: 10.1186/1475-2867-9-14
- YIJUN LIU ET AL: "Metabolic regulation of mesenchymal stem cell in expansion and therapeutic application", BIOTECHNOLOGY PROGRESS., vol. 31, no. 2, 20 December 2014 (2014-12-20), pages 468-481, XP055377111, US ISSN: 8756-7938, DOI: 10.1002/btpr.2034
- ADRIEN MOYA ET AL: "Quiescence Preconditioned Human Multipotent Stromal Cells Adopt a Metabolic Profile Favorable for Enhanced Survival under Ischemia : Quiescence Preconditioning Enhances hMSC Survival", STEM CELLS., vol. 35, no. 1, 21 September 2016 (2016-09-21), pages 181-196, XP055377118, US ISSN: 1066-5099, DOI: 10.1002/stem.2493
- MARY BOARD ET AL: "Acetoacetate is a more efficient energy-yielding substrate for human mesenchymal stem cells than glucose and generates fewer reactive oxygen species", INTERNATIONAL JOURNAL OF BIOCHEMISTRY AND CELL BIOLOGY, vol. 88, 5 May 2017 (2017-05-05), pages 75-83, XP055377126, GB ISSN: 1357-2725, DOI: 10.1016/j.biocel.2017.05.007

## Description

### Background of the invention

The invention relates to a method and culture medium for cultivating primary cells *in vitro.*

The potential for cell therapy has received a great deal of popular and scientific attention over recent years with proposals that transplantation of cells could take the form of an acute treatment for myocardial infarction, for example, or a chronic treatment for neurodegenerative disorders. Any form of therapy involves the extraction, storage and *in vitro* culture of primary cells raising the possibility that these processes, in particular *in vitro* culture, would have an impact on the characteristics of the cells. Both glucose and glutamine routinely included in growth medium can be used by these cells to generate ATP and pyruvate, where present, may also fulfil this role. Accordingly, cells in culture are normally exposed to glucose and glutamine and, occasionally pyruvate, in their growth media. However, a wider range of energy-yielding substrates may be available to the cells *in vivo.* It is therefore desirable to provide additional, and at best more effective, potential energy-yielding substrates contributing to ATP-synthesis in primary cells growing in culture.

### Summary of the invention

It is the object of the invention to provide a method and culture medium for cultivating primary cells *in vitro,* which comprise more effective energy-yielding substrates and represent advantageous modifications to primary cell culture.

The object is met by a method for cultivating primary cells *in vitro,* wherein said primary cells are Mesenchymal Stem Cells (MSCs), and wherein the cells are cultivated in a culture medium comprising at least one ketone body. Surprisingly, a high rate of ketone body oxidation can be observed at all passage numbers in *in vitro* culture under various culture conditions. Not only does this indicate that the cells have a functional TCA cycle of a higher capacity than would be deduced from their oxidation of glucose, but it also highlights the effectiveness of ketone bodies as a potential energy-yielding substrate. This observation has obvious implications for the design of culture medium for the culture of Mesenchymal Stem Cells (MSCs). Glucose and glutamine, the usual substrates included in standard growth medium, are not as effective in producing ATP and thus the design of a medium incorporating a ketone body stimulates energy-production by these cells. Moreover, it was observed that rates of reactive oxygen species (ROS) generation are significantly lower, on a mole ROS per mole substrate oxidised basis, with a ketone body substrate than they are with a glucose substrate. This may partly explain why oxidation of ketone bodies represents an appropriate metabolic strategy for these cells and may indicate a preference for an acetoacetate substrate when a range of substrates are available.

In an advantageous embodiment of the invention the cells are cultivated in a gas atmosphere comprising an oxygen concentration of about 10 - 30 % by volume, preferably 15 - 25 % or 19 - 22 % by volume, in particular 20 - 21 % by volume. Such oxygen concentrations represent slightly hypoxic to normoxic conditions, or may be even higher (if suitable). Given the suggestions that stem cells *in vivo* occupy hypoxic niches (Schofield, 1978; Crisan et al., 2008; Parmar et al, 2007; Suda et al., 2011) and that their energy-yielding metabolism is likely to be correspondingly hypoxic and to include a reliance on the pathway of glycolysis for ATP-generation (Pattappa et al., 2011; Simsek et al., 2010; reviewed by Gaspar et al., 2014), it is surprising that normoxic culture of Mesenchymal Stem Cells (MSCs) results in a general increase in flux through energy-yielding pathways and a rise in substrate oxidation rates. This implies that energy-yielding pathways are generally upregulated for cells cultured *in vitro* under normoxic conditions but not for cells cultured under hypoxic conditions.

In another advantageous embodiment of the invention the ketone body is selected from the group consisting of acetoacetate (3-oxobutyrate), *beta-*hydroxybutyrate (3-hydroxybutyrate), *beta*-ketopentanoate (3-oxopentanoate), and *beta*-hydroxypentanoate (3- hydroxypentanoate).

In another advantageous embodiment of the invention the culture medium further comprises pyruvate. Surprisingly, the combination of a ketone body (e.g. acetoacetate) with pyruvate proved to be the most successful in terms of calculated ATP-yield. A culture medium incorporating both a ketone body and pyruvate is therefore the most effective substrate to stimulate energy-production in Mesenchymal Stem Cells (MSCs).

In a further advantageous embodiment of the invention the culture medium is devoid of glucose or any precursor thereof. As the rate of ketone body oxidation is significantly reduced by the addition of glucose and thus the presence of glucose seems to have a suppressing effect on ketone body utilisation, it is advantageous that the Mesenchymal Stem Cells (MSCs) are cultured in the absence of glucose or any precursor thereof. In this context, "precursor" as used herein refers to molecules or compositions from which glucose can be obtained by hydrolysis, including but not limited to carbohydrates such as milk sugar, cane sugar, maltose, cellulose, glycogen, starch etc.

In a further advantageous embodiment of the invention said Mesenchymal Stem Cells (MSCs)are human Mesenchymal Stem Cells (hMSCs).

The object is further met by the use of a culture medium comprising at least one ketone body for cultivating Mesenchymal Stem Cells (MSCs) *in vitro.* As glucose and glutamine, the usual substrates included in standard culture medium for Mesenchymal Stem Cells (MSCs), are not as effective in producing ATP as a ketone body, providing a culture medium comprising a ketone body instead of the usually used substrates significantly enhances energy-production by these cells. Moreover, the rates of generation of reactive oxygen species (ROS) are significantly lower, on a mol ROS per mol substrate oxidised basis, with a ketone body substrate than they are with a glucose substrate. Accordingly, ketone bodies represent a more effective and beneficial energy-yielding substrate in a culture medium for cultivating Mesenchymal Stem Cells (MSCs).

In an advantageous embodiment of the invention the ketone body is selected from the group consisting of acetoacetate (3-oxobutyrate), *beta*-hydroxybutyrate (3- hydroxybutyrate), *beta*-ketopentanoate (3-oxopentanoate), and *beta-*hydroxypentanoate (3- hydroxypentanoate).

The culture medium for use in cultivating Mesenchymal Stem Cells (MSCs)may further comprise pyruvate. The combination of a ketone body (e.g. acetoacetate) with pyruvate proved to be the most successful in terms of calculated ATP-yield so that a culture medium incorporating both a ketone body and pyruvate is the most effective substrate to stimulate energy-production in Mesenchymal Stem Cells (MSCs).

As the rate of ketone body oxidation is significantly reduced by the addition of glucose and thus the presence of glucose seems to have a suppressing effect on ketone body utilisation, it is advantageous if the culture medium is devoid of glucose or any precursor thereof. In this context, "precursor" as used herein refers to molecules or compositions from which glucose can be obtained by hydrolysis, including but not limited to carbohydrates such as milk sugar, cane sugar, maltose, cellulose, glycogen, starch etc.

In another advantageous embodiment of the invention the culture medium for use in cultivating Mesenchymal Stem Cells (MSCs) further comprises a base medium such as Dulbecco's Modified Eagle's medium (DMEM) or or any other suitable medium for culturing Mesenchymal Stem Cells (MSCs) *in vitro.* Optionally, the culture medium for use in cultivating Mesenchymal Stem Cells (MSCs) may further comprise at least one supplement, for example, glutamine and or an antibiotic substance such as gentamycin.

Culture of human mesenchymal stem cells (hMSCs) is vital for therapeutic exploitation. These cells occupy hypoxic niches *in vivo* but more ATP was consistently produced from substrate-oxidation than glycolysis by cultured hMSCs. Strong substrate preferences were shown with the ketone body, acetoacetate, being oxidised at up to 42 times the rate of glucose. Acetoacetate oxidation reduced generation of reactive oxygen species (ROS) 4.5-fold compared with glucose and substrate preference may be an adaptation to reduce oxidative stress. The UCP2-inhibitor, genipin, increased ROS production with acetoacetate (2-fold) and glucose (1.6-fold), indicating a role for UCP2 in suppressing ROS-production. Addition of pyruvate stimulated acetoacetate-oxidation and this combination increased ATP-production 28-fold, compared with glucose alone, which has implications for growth medium composition. Oxygen tension affected metabolism by hMSCs. Between passages 2 and 5, rates of both glycolysis and substrate oxidation increased at least 2-fold for normoxic (20% O₂) - but not hypoxic (5% O₂) - cultured hMSCs, despite declining growth rates and no detectable signs of differentiation. These findings have implications for stem cell therapy since low passage number normoxic-cultured stem cells show metabolic adaptations without detectable changes in stem-like status.

"Primary cell" as used herein refers to a cell taken directly from living tissue (e.g. biopsy material, blood sample or the like) to be established for growth in *in vitro* culture. Primary cells represent a more representative model to the *in vivo* state as they have undergone only very few passages (population doublings) and are therefore more representative of the main functional components of the tissue from which they derived in comparison to continuous (tumor or artificially immortalized) cell lines.

"Stem cell" as used herein refers to an undifferentiated biological cell that can differentiate into specialized cells and can divide to produce more stem cells. In contrast to embryonic stem cells, which can be solated from the inner cell mass of blastocysts, it is preferred to use adult stem cells which are found in various tissues such as bone marrow, adipose tissue (lipid cells), and peripheral blood or umbilical cord blood.

"Ketone body" as used herein refers to a water-soluble molecule that is produced by the liver from fatty acids during periods of low food intake, starvation, prolonged intense exercise, or in un- or inadequately treated type 1 diabetes mellitus. Ketone bodies are readily picked up by the extra-hepatic tissues and converted into acetyl-CoA which then enters the citric acid cycle and is oxidized in the mitochondria for energy. Besides the endogenous ketone bodies (acetoacetic acid, *beta*-hydroxybutyric acid, and acetone) other ketone bodies such as beta-ketopentanoate and beta-hydroxypentanoate can be created as a result of the metabolism of synthetic triglycerides, e.g., triheptanoin.

"Normoxic" as used herein refers to a gas atmosphere comprising an oxygen concentration of 19 - 22 % by volume, in particular 20 - 21 % by volume. An oxygen concentration of about 21 % by volume correlates to an O₂ partial pressure (pO₂) of about 21 kPa (0.21 bar). Physiologically, pO₂ is much lower, 1-2 orders of magnitude. The often used term "normoxic" refers to the artificial pO₂ in cell culture incubators which are, for convenience, using air enriched with CO₂, hence have about 0.2 bar pO₂.

"Hypoxic" as used herein refers to a gas atmosphere comprising an oxygen concentration of about 0.01 - 15 % by volume, in particular 0.1 - 10 or 1 - 9 % by volume, for example 4 - 6 % by volume.

The invention is further described in detail with reference to the figures and tables.

### Brief description of the figures

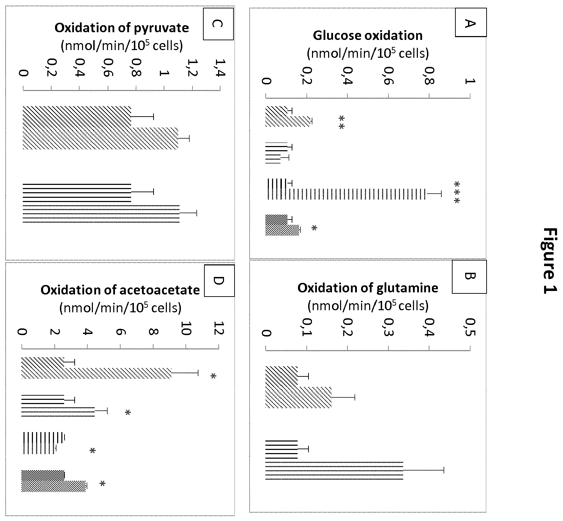

**Figure 1** **shows bar diagrams representing the oxidation of energy-yielding substrates by hMSCs.**
   Rates of oxidation of 5.5mM glucose (A); 1mM glutamine (B); 2mM pyruvate (C), and 10mM acetoacetate (D) are shown for cells at passage 2 (black) and passage 5 (red). Rates of substrate oxidation were measured in 24-well plates as the production of ¹⁴CO₂ from ¹⁴C-labelled substrates. Cells were cultured as follows through passages 1 to 5: normoxia (20% O₂, cross-hatched); hypoxia (5% O₂, vertical lines); normoxia with 5mM 3-hydroxybutyrate (3-HB), horizontal lines); hypoxia with 5mM 3-HB, stippled). n≥5 for each column.
   * : p < 0.05, ** p < 0.01 and ***p<0.0001 when substrate oxidation at passage 5 (p5) is compared with that at passage 2 (p2).

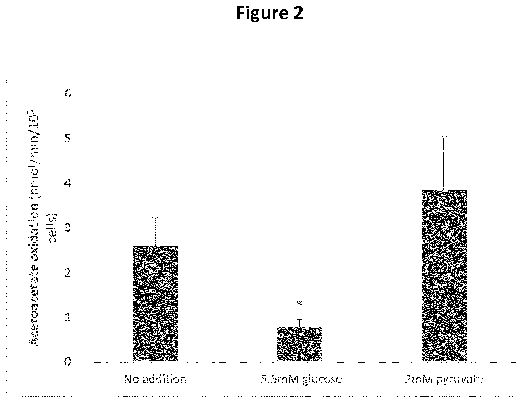

**Figure 2** **shows a bar diagram representing the oxidation of acetoacetate by hMSCs: effects of additional substrates.**
   Rates of oxidation of 10mM acetoacetate containing a trace of 3-¹⁴C-acetoacetate were measured in the presence of unlabelled 5.5mM glucose or 2mM pyruvate, where marked. Oxidation was measured in 24-well plates. Cells were passage 2, cultured under normoxic conditions.
   * : p = 0.0247 compared with acetoacetate alone.

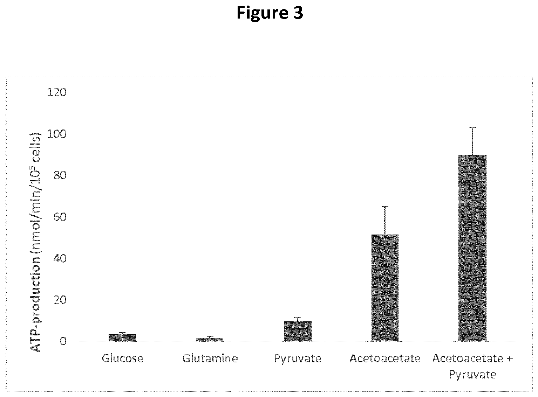

**Figure 3** **shows a bar diagram representing the calculated rates of ATP-production by hMSCs with oxidative substrates.**
   Rates of ATP-production from individual substrates were calculated on the basis of mol ATP produced /mol substrate oxidised. Assuming full oxidation of each mole of substrate converted to CO_{2.}, it is calculated that 1 mole of glucose will produce a mean value of 31 moles ATP; glutamine: 20; pyruvate: 12.5; acetoacetate: 20 (refer to Experimental Procedures for complete explanation). For calculations of ATP-production from acetoacetate plus pyruvate, separate incubations were performed with 10mM acetoacetate labelled with ¹⁴C-acetoacetate in the presence of unlabelled 2mM pyruvate and with 2mM pyruvate labelled with ¹⁴C-pyruvate in the presence of unlabelled 10mM acetoacetate. Rates of ATP-production from the labelled substrate in each incubation were calculated and the results summed. Cells were passage 2, cultured under normoxic conditions.

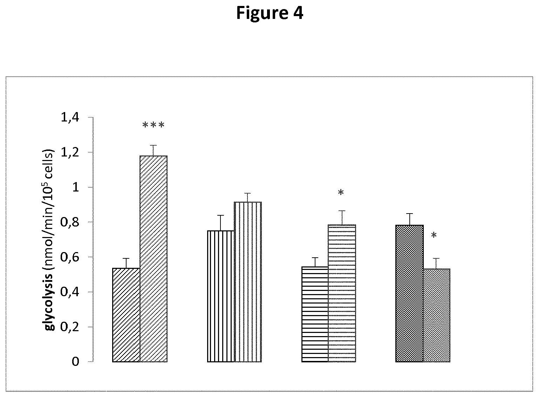

**Figure 4** **shows a bar diagram representing the rates of glycolysis by hMSCs at passages 2 and 5.**
   Rates of glycolysis were measured for hMSCs at passages 2 (black) and 5 (red) when cultured as follows through passages 1 to 5: normoxia (20% O₂, cross-hatched); hypoxia (5% O₂, vertical lines); normoxia with 5mM 3-HB (horizontal lines); hypoxia with 5mM 3-HB (stippled).
   Glycolytic rates were measured in 24-well plates by the method outlined in the Experimental Procedures section.
   *** : p<0.0001 compared with normoxic cells at passage 2;
   * p <0.04 compared with cells cultured under the same conditions at passage 2.

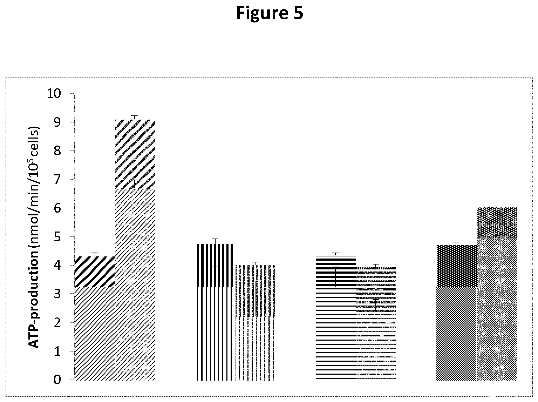

**Figure 5** **shows a bar diagram representing the calculated rates of ATP-production by hMSCs from glycolysis and from full oxidation of glucose.**
   Rates of ATP-production were calculated as previously (refer to Experimental Procedures section for full explanation). Cells were cultured under normoxic conditions (20% O₂, cross-hatched); hypoxic (5% O₂, vertical lines); normoxic with 5mM 3-HB (horizontal lines); hypoxic with 5mM 3-HB (stippled) though passages 1 to 5. ATP-production from full oxidation of ¹⁴C-labelled substrates is shown in the lower column of each data point (light patterning) and that from glycolysis is shown in the upper column (heavy patterning). Measurements were made at passage 2 (black) and passage 5 (red) for each culture condition. n≥9.

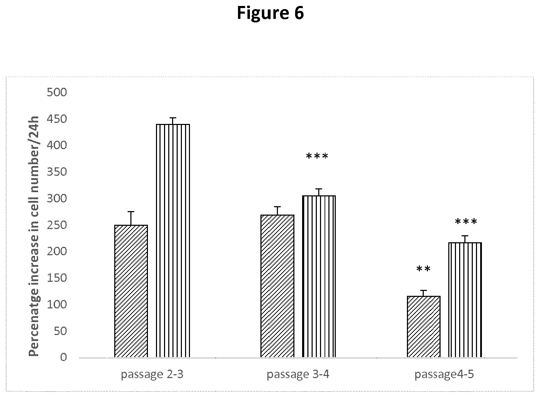

**Figure 6** **shows a bar diagram representing the growth rates of hMSCs with passage number.**
   HMSCs were cultured under normoxic (20% O₂, cross-hatched) or hypoxic (5% O₂, vertical lines) conditions. Cells were passaged every 48h and cell numbers counted by haemocytometer, using Trypan Blue to assess viability.
   n=4
   **: P=0.0025 compared with passage 2-3;
   *** p<0.0003 compared with passage 2-3.

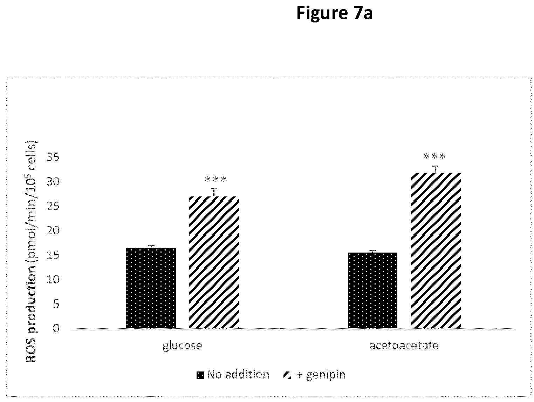

**Figure 7a** **shows a bar diagram representing the production of ROS by hMSCs with glucose and acetoacetate : effects of genipin.**
   hMSCs were cultured under normoxic conditions and were at passage 5 at the time of assay. For measurements in the presence of genipin, cells were pre-incubated for 24h with 20µM genipin. Cells were incubated for 90 minutes in 24 well plates in the presence of either 5.5mM glucose or 10mM acetoacetate before measurement of ROS. Values were calculated for 6 replicates.
   *** : P<0.0001 for conditions with genipin compared with no addition.

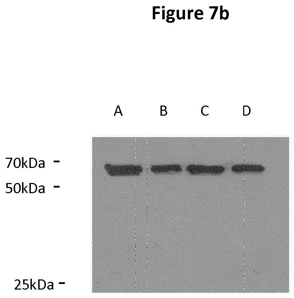

**Figure 7b** **shows a picture of a Western Blot of hMSCs with anti-UCP2 antibody.**
   Cell extracts were probed with anti-human UCP2 antibody and the probe detected using a secondary anti-rabbit antibody. Cells were cultured as follows: Lanes A and B under normoxic conditions and lanes C and D under hypoxic conditions. The presence of UCP2 protein was detected at passages 2 (lanes A and C) and 5 (lanes B and D).

### Description of exemplary and preferred embodiments of the invention

### Experimental Procedures:

### Human Mesenchymal Stem Cells

Stem cells were obtained from 3 sources: a generous gift from Dr. Yasser El-Sherbini, Dept. of Biomedical Engineering, University of Oxford, and purchased from Millipore.

### Culture of cells

Cryopreserved cells were thawed, washed with growth medium and seeded at a density of 1 X 106 cells per 225cm2 with Dulbecco's Modified Eagle's medium (DMEM) containing 2mM ultra-glutamine, 1g/l glucose, 50µg/ml gentamycin and 10% FBS. Where 3-hydroxybutyrate (3-HB) was included in the growth medium, the concentration was 5mM throughout. Cells were passaged at about 80% confluence. Cells for measurements of glycolysis or oxidation were seeded into 24-well plates at a density of 1 x 10⁴ cells per well and used at about 90% confluence. Cells were maintained in either normoxic culture with 20% O₂ tension or hypoxic culture with 5% O₂ tension in an oxygen-tension controlled incubator. Previous workers have identified 5% O₂ as being a physiologically significant level of O₂ with respect to *in vivo* conditions for hMSCs, representing hypoxia (Pattappa et al., 2011).

### Measurements of substrate-oxidation in 24-well plates

Cells were incubated for 90 minutes in the presence of a single radiolabelled substrate (with addition of unlabelled substrates where indicated) in DMEM (containing no pyruvate, glucose or glutamine) in a total volume of 0.5 mls. The concentrations of single substrates were: 5.5mM glucose (considered similar to median human blood glucose concentration); 2mM pyruvate (considered higher than endogenous pyruvate concentration from glucose metabolism and sufficient to stimulate activity of pyruvate dehydrogenase plus pyruvate carboxylase); 1mM glutamine (considered similar to human blood glutamine concentrations); 10mM acetoacetate (considered similar to intracellular acetoacetate concentrations produced from maximal blood ketone body concentrations). In addition, substrates incorporated radiolabels as follows: glucose: 21 MBq/mmol D-U-¹⁴C-glucose; pyruvate: 0.35MBq/mmol 1-¹⁴C-pyruvate; acetoacetate: 0.185MBq/mmol 3-¹⁴C-acetoacetate; glutamine: 1.2 MBq/mmol U-¹⁴C-glutamine. Evolved ¹⁴CO₂ was trapped and measured by the method of Collins et al. (1998), except that, after addition of perchloric acid to the wells, plates were gently agitated for 60 minutes to release dissolved ¹⁴CO₂. Filter papers containing trapped ¹⁴CO₂ were counted in Ecoscint using a Tri-Carb 2800TR Liquid Scintillation Analyser. Preliminary measurements showed that rates of ¹⁴CO₂-production were linear for a period of at least 120 minutes under these conditions. Rates of ATP-production from individual substrates were calculated on the basis of mol/mol production as follows: glucose: 31; glutamine: 20; pyruvate: 12.5; acetoacetate: 20. For calculations of ATP-production from acetoacetate plus pyruvate, separate incubations were performed with ¹⁴C-acetoacetate in the presence of unlabelled pyruvate and with ¹⁴C-pyruvate in the presence of unlabelled acetoacetate. Rates of ATP-production from the labelled substrate were calculated for each condition and the results summed.

### Measurements of glycolytic rate in 24 well plates

Glycolysis was measured as the production of ³H₂O from 5-³H-glucose, which occurs during the step catalysed by enolase. Cells were incubated with 5.5mM glucose containing 17 MBq/mmol 5-³H-glucose in DMEM without pyruvate, glutamine or glucose for 90 minutes in a total volume of 0.5mls. At 90 minutes, 0.2 mls of the medium was applied to a column (volume 1ml) of Dowex-1-borate (prepared from Dowex-1-chloride by the method of Hammerstedt, 1973). Washing with 2 column volumes of water eluted the ³H₂O which was counted for radioactivity (Tri-Carb 2800TR Liquid Scintillation Analyser).

### Calculation of ATP-yield

ATP-yields were calculated from the following values, as follows: from glycolysis of glucose, it is assumed that 2 moles ATP will be produced per mole glucose glycolysed. For substrate-oxidation measurements, it is assumed that full oxidation of each substrate takes place. Under these conditions, glucose oxidation will produce a mean value of 31 mol ATP/mol glucose when glucose is converted to CO₂ by the action of glycolysis, the pyruvate dehydrogenase reaction and reactions of the tricarboxylic acid cycle. It is assumed that cytosolic NADH generated from glycolysis will be re-oxidised due to the action of either the 3-phosphoglycerate or the malate-aspartate shuttles. Pyruvate oxidation generates 12.5 mol ATP/mol pyruvate when the substrate is fully converted to CO₂ by the actions of the pyruvate dehydrogenase reaction and reactions of the tricarboxylic acid cycle. Acetoacetate oxidation generates 20 mol ATP/mol acetoacetate when the substrate is first split into acetyl coenzyme A which is fully converted to CO₂ by reactions of the tricarboxylic acid cycle. Glutamine oxidation generates 20 mol ATP/mol glutamine when the substrate is deaminated to α-ketoglutarate and α-ketoglutarate converted to pyruvate by reactions of the tricarboxylic acid cycle plus that of pyruvate carboxylase. The resulting pyruvate is then oxidised in the same way as exogenous pyruvate.

### Measurement of Reactive Oxygen Species

Cells were incubated under appropriate conditions in 24-well plates in a total volume of 0.2ml for 90 minutes. At 90 minutes, ROS were assayed using an Oxiselect Amplex Red kit from Cell Biolabs, Inc., according to the manufacturer's instructions. Where genipin was used, cells were pre-incubated for 24 hours with 20 µM genipin.

### Growth rates of hMSCs

At each passage (typically every 48h), cell numbers were counted in a haemocytometer using Trypan Blue exclusion as a gauge of viability.

### qPCR

Expression of marker proteins (MSC markers: CD73, CD105, Stro1, CD44, CD146, CD90; markers of pluripotency: Nanog, Oct4, cMyc, Sox2, TERT, PPARγ; markers of differentiated lineages: Osteopontin, BMP2, CD45, CD11b, CD14, Notch1) was assessed by qPCR. Cells of the appropriate passage number were pelleted and RNA extracted by anion exchange (Qiagen RNeasy Mini kit). The purity was assessed and the RNA quantified by nanospectrophotometer (Nanodrop Lite spectrophotometer). Synthesis of cDNA was achieved using an Applied Biosystems High Capacity cDNA Reverse Transcription Kit. Forward and reverse primers used to amplify target loci were, respectively:
CD73: CGCAACAATGGCACAATTAC (SEQ ID NO: 1);
CTCGACACTTGGTGCAAGA (SEQ ID NO: 2) (Rada et al. 2011);
CD105: TCCTCCCAAGGACACTTGTA (SEQ ID NO: 3);
CGCCTCATTGCTGATCATAC (SEQ ID NO: 4) (Rada et al. 2011);
Stro1: GAAGCTAAAGTGGATTCAGGAGTA (SEQ ID NO: 5);TAAGCAGGGGACCATTACA (SEQ ID NO: 6) (Rada et al., 2011);
CD44: GTTGCCAAACCACTGTTCCT (SEQ ID NO: 7);
CATTCAAATCCGGAATGCT (SEQ ID NO: 8) (Rada et al. 2011);
CD146: CCAAGGCAACCTCAGCCATGTC (SEQ ID NO: 9);
CTCGACTCCACAGTCTCCCACGACT (SEQ ID NO: 10) (Furstenberger et al. 2005);
CD90: GACAGCCTGAGAGGGTCTTG (SEQ ID NO: 11);
CCCAGTGAAGATGCAGGTTT (SEQ ID NO: 12) (Li et al, 2013);
Nanog: ACCAGAACTGTGTGTTCTCTTCCACC (SEQ ID NO: 13);
CCATTGCTATTCTTCGGCCAGTTG (SEQ ID NO: 14) (Wilson et al. , 2010);
Oct4: GCTCGAGAAGGATGTGGTC (SEQ ID NO: 15);
ATCCTCTCGTTGTGCATAGTCG (SEQ ID NO: 16) (Liu et al. 2011);
cMyc: CGTCTCCACACATCAGCACAA (SEQ ID NO: 17);
TCTTGGCAGCAGGATAGTCCTT (SEQ ID NO: 18) (Liu et al, 2011);
Sox2: CACTGTCCCTCTCACACATG (SEQ ID NO: 19);
CCCATTTCCCTCGTTTTTCTT (SEQ ID NO: 20) (Liu et al., 2011);
TERT: GAGCTGACGTGGAAGATGAG (SEQ ID NO: 21);
CTTCAAGTGCTGTCTCTGATTCCAATG (SEQ ID NO: 22) (Friedrich et al, 2004);
PPARγ: GGCTTCATGACAAGGGAGTTTC (SEQ ID NO: 23);
AACTCAAACTTGGGCTCCATAAG (SEQ ID NO: 24) (Zhang et al. 2007);
Osteopontin: CCCACAGACCCTTCCAAGTA (SEQ ID NO: 25);
GGGGACAACTGGAGTGAAAA (SEQ ID NO: 26) (Rada et al., 2011);
BMP2: TCCTCTCATCAGCCATTTGTCCTTTC (SEQ ID NO: 27);
AGTTACTACACATTCTTCATAG (SEQ ID NO: 28) (Pera et al., 2004);
CD45: ACGAAGCTCTTAGCGTCAGG (SEQ ID NO: 29);
CTCTCGGGTGGAGTCTTCTG (SEQ ID NO: 30) (Li et al, 2013);
CD11b: AGCCCAAGATCACATG (SEQ ID NO: 31); TGCAGAAGCATAACCC (SEQ ID NO: 32) (Zhou et al., 2005);
CD14: GACTTATCGACCATGGAGCG (SEQ ID NO: 33);
CCAGTAGCTGAGCAGGAACC (SEQ ID NO: 34) (Pilz et al., 2011);
Notch1: GACTATGCCTGCAGCTGTGCC (SEQ ID NO: 35);
GGCTGCAGGGCACGTAGG (SEQ ID NO: 36) (Okumoto et al, 2003).
cDNA was amplified during 40 cycles of PCR and quantified by Syb R-based fluorescence (Primer Design Precision FAST qPCR Mastermix). Gene expression at passage 5 compared with passage 2 was quantified by the 2^{-ΔΔCT} method (Livak and Schmittgen, 2001) relative to housekeeping genes (GAPDH; RNApolll;) at the same passage numbers.

### Detection of UCP2 by Western Blotting

Western blots were performed under denaturing conditions. Cells were detached using trypsin, homogenised using a 21G needle, incubated for 5 minutes in a dri-block at 100° C and centrifuged at 13,000rpm for 10 minutes. Aliquots of the supernatant were assayed for protein concentration (Pierce BCA protein assay kit) and the remainder had 5% mercaptoethanol added before incubation for 5 minutes at 100° C. Blots were performed by the method of Heather et al (2011), where equal quantities of total cellular protein (25 µg) were loaded per well, proteins were stained with Ponceau's reagent and gels blocked with 5% milk powder. UCP2 protein was detected using rabbit polyclonal anti-human UCP2 antibody (5µg/ml) purchased from Abcam and donkey anti-rabbit secondary antibody allowed detection by exposure to X-ray film. Rainbow marker proteins (25-120 kDa) were used to assess molecular weight.

### Differentiation of hMSCs

Cells at passage 6 were seeded at a density of 2x10⁵ (adipogenesis and chondrogenesis) or 3x10⁴ (osteogenesis) into wells of a 6 well tissue culture plate. Kits for differentiation along adipogenic, chondrocytic and osteocytic lineages were obtained from Lonza and accompanying protocols followed. Early stage characteristics of differentiation were then assessed. Adipocytes were assayed for fat accumulation after 18 days from the onset of the differentiation protocol. Cells were fixed by incubating for 10 minutes in 10% formalin, followed by 60 minutes in fresh 10% formalin and washing with 60% isopropanol. 5mM Oil red O in 60% isopropanol (filtered), 1ml per well, was added to dry wells and left for 10 minutes before washing four times with water. Oil red O was eluted with 1ml 100% isopropanol per well and the optical density measured at 500nm. Chondrocytes were assayed for collagen accumulation (after 21 days from the onset of the differentiation protocol) using Sirius red/fast green collagen staining kit purchased from Amsbio. Osteocytes were assayed for calcium accumulation (21 days after the onset of the differentiation protocol) by the method of Jager et al, 2005. In each case, values were compared with those of control cells which had been cultured under normal growth conditions without being exposed to differentiation protocols.

### Data analysis

The significance of differences in pairs of means was assessed statistically, using Student's t test by Graphpad Quickcalcs online analysis for continuous data.

### Results:

### Oxidation of glucose, pyruvate, glutamine and acetoacetate

Oxidation rates for both glucose and acetoacetate rise with passage number for normoxic-cultured cells (2-fold increase in glucose-oxidation rate and 3.5-fold increase in acetoacetate-oxidation rate) between passage 2 and passage 5 (p2-p5) whereas rates of oxidation of pyruvate and glutamine are not significantly different at the two passage numbers **(****Fig.1 A). Figure 1** shows that acetoacetate is oxidised at a 25-fold higher rate than glucose at p2 and this rises to a 42-fold higher rate at p5. This increase is not seen for hypoxic-cultured cells, where the rate of oxidation of neither substrate is significantly different at p5 compared with p2. Pyruvate and acetoacetate, which both feed directly into the mitochondrial TCA cycle, show the highest rates of oxidation of all substrates for all culture conditions which would seem to indicate the functionality of the TCA cycle in these cells. When cells were cultured in the presence of 5mM 3-hydroxybutyrate (3-HB) from passages 1 to 5, the increase in oxidation rate at passage 5 for normoxic cells was greater (7.5-fold) for glucose but was reduced by 1.3-fold for acetoacetate, giving an oxidation profile which more nearly resembled the 3-HB-free hypoxic condition than the 3-HB-free normoxic.

### Oxidation of acetoacetate : effects of additional substrates

Adding glucose to acetoacetate oxidation experiments reduces the rate of acetoacetate-oxidation by more than 3-fold **(****Fig. 2****)** for normoxic-cultured cells, suggesting either that the presence of glucose has a suppressing effect on acetoacetate utilisation or that pyruvate from glucose competes with acetyl CoA from acetoacetate for oxidation by the TCA cycle. However, addition of pyruvate does not result in a significant difference in rates of acetoacetate oxidation so that competition seems unlikely. Indeed, it could be that the pyruvate substrate is carboxylated to oxaloacetate as a result of intramitochondrial pyruvate carboxylase activity (as well as being decarboxylated to acetyl Coenzyme A by pyruvate dehydrogenase) in these cells, so that the presence of pyruvate may maintain the capacity of the TCA cycle enabling continued acetoacetate oxidation. This action would explain the small, non-significant increase in acetoacetate-oxidation rates seen when pyruvate is present **(****Fig. 2****).** Addition of malate had no significant effect on rates of pyruvate oxidation (data not shown), indicating that carboxylation of pyruvate is not the only route for oxidative metabolism of this substrate. This may explain the difference observed between the effects of glucose and those of pyruvate on acetoacetate oxidation, despite the fact that both substrates would be expected to give rise to intramitochondrial pyruvate. The higher concentrations of intramitochondrial pyruvate achieved when 2mM pyruvate is the substrate may lead to appreciable activity of pyruvate carboxylase, while lower concentrations of pyruvate from glucose may not.

### ATP-production from substrate-oxidation

Calculated rates of ATP-production demonstrate that acetoacetate is the single most efficient substrate for ATP-production by these cells, when full oxidation of the substrate is assumed **(****Fig. 3****).** Neither glucose nor glutamine, the usual additions to tissue culture medium, is as productive of ATP (glucose produces 16 times less ATP than acetoacetate alone and glutamine produces 33 times less). The presence of pyruvate increases the yield of ATP from acetoacetate, suggesting that this would be the most productive substrate combination for energy-yield by these cells.

### Anaerobic metabolism of glucose : rates of glycolysis

Glycolytic rates are not significantly different for cells cultured under normoxic and hypoxic conditions at passage 2 **(****Fig. 4****).** However, more unexpected is the 2.2-fold rise in glycolytic rates seen for cells cultured under normoxia between passages 2 and 5. This effect is not seen for cells cultured under hypoxia and rates of glycolysis for hypoxic p5 cells are 23% lower than those for normoxic cultured cells. This mirrors the rise in oxidation rates seen for normoxic cultured cells **(****Fig 1****)** and implies that energy-yielding pathways are generally upregulated between the two passage numbers for cells cultured under 20% oxygen tension but not for cells cultured under 5% O₂ tension. When the cells were cultured with 3-HB, an increase in glycolytic rate at p5 is again seen for normoxic cultured cells but is reduced to 1.4-fold. For cells cultured under hypoxia with 3-HB, glycolytic rates are significantly decreased by passage 5 (1.5-fold). The presence of 3-HB thus seems to have a suppressing effect on the increase in energy-yielding metabolism which follows from normoxic culture for both aerobic and anaerobic pathways **(****Figs. 1** **and** **4****).** The energy profile of cells cultured in the presence of 3-HB and 20% O₂ thus mimics that of cells cultured under hypoxic conditions.

### ATP-production from anaerobic and aerobic metabolism of glucose

**Figure 5** shows that normoxic-cultured cells increase their calculated ATP-production from both aerobic and anaerobic metabolism of glucose by a factor of 2 between passages 2 and 5. Interestingly, the proportion of ATP coming from each branch of metabolism remains constant (75% from glucose oxidation and 25% from glycolysis). Hypoxic cultured cells do not show the same increased rate of ATP-generation and the inclusion of 3-HB in the culture medium of normoxic cells also reduces the passage-related increase in ATP-generation producing a profile that resembles more closely that of hypoxic cultured cells than of normoxic. It would seem likely that the normoxic-cultured cells increase their rate of ATP-production either because they show increased rates of growth between passages 2 and 5 or because the cells are synthesising macromolecules in preparation for differentiation.

### Growth rates of hypoxic- and normoxic-cultured cells between passages 2 and 5

**Figure 6** demonstrates that growth rates decline with passage number for both normoxic and hypoxic culture conditions so that the cells are dividing at lower rates by passage 5 than at passage 2. This observation is true both for the normoxic-cultured cells, which accelerate their rates of ATP-production, and for the hypoxic-cultured cells which do not.

### Production of mRNA for markers of stem-like. hMSC and differentiated status

Markers of MSCs, general stem-like status or differentiation do not change for normoxic cultured cells between passage 2 and 5 **(Table S1).** Interestingly, the only changes are seen during hypoxic culture, where the cells show increased production of mRNA for the general markers of the stem-like state, Oct4, c-myc and Sox2 and of the osteocyte-specific protein, osteopontin. None of the other markers of osteocytes show similar changes. For the cells of the present study, there are no clear indications that differentiation is underway by passage 5.

### Production of ROS by hMSCs incubated with glucose and acetoacetate

In order to investigate whether substrate-availability has an influence on oxidative stress in hMSCs, levels of ROS were measured after incubation with either a glucose or an acetoacetate substrate. **Figure 7a** shows rates of ROS-production with these two substrates. If the ROS-production is considered relative to rates of substrate oxidation, then each mole of glucose oxidised generates 0.076 moles of ROS whereas each mole of acetoacetate oxidised generates 0.0017 moles of ROS, a 4.5-fold difference. It has been suggested that the activity of UCP2 may influence the production of ROS (Negre-Salvayre et al., 1997; Arsenijevic et al, 2000). Effects of the specific UCP-2 inhibitor, genipin (Zhang et al., 2006), indicate that this observation may be true for the hMSCs of the present study. The presence of genipin results in a 1.6-fold increase in ROS-production with a glucose substrate and a 2-fold increase when acetoacetate is the substrate.

### Detection of human UCP2 by Western blotting

The results of a Western blot using anti-human UCP2 antibody are shown in **Figure 7b****.** Cells cultured under all conditions show the presence of an approximately 66kDa protein recognised by the antibody. The active UCP2 protein is a dimer composed of two subunits of approximately 32-35kDa (reviewed by Ledesma et al., 2002). No band corresponding to the monomer is seen in the cells of the present study. It has been shown for other members of the anion carrier superfamily that the two subunits may be covalently linked to form a functional unit (Trézéguet et al. , 2000).

### Differentiation of hMSCs along adipocytic, chondrocytic and osteocytic lineages

Cells grown under all conditions were assessed for their ability to differentiate at passage 6. All cells were successfully differentiated into adipocytes, chondrocytes and osteocytes and accumulation of intracellular fat, collagen and calcium were measured **(Table S2).** *Adipocyte differentiation:* intracellular fat accumulation was measured by Oil red O staining and differentiated cells showed a mean 30% increase over control cells which were cultured as normal and not subjected to the differentiation protocol. *Chondrocyte differentiation:* production of collagen (assessed by Sirius red/fast green staining) showed a mean 45% increase over control cells. *Osteocyte differentiation:* intracellular calcium accumulated to a mean 44% increase over control cells for cells cultured under hypoxic conditions but to a mean 26% increase for cells cultured under normoxia. There were no significant effects of culture conditions, except in the case of osteocyte differentiation stated above. Thus, hMSCs cultured under all conditions used in the present study retain the capacity to differentiate along all three expected lineages.

A salient characteristic of hMSC metabolism is the high rate of acetoacetate oxidation seen at all passage numbers and under both normoxic and hypoxic culture. Not only does this indicate that the cells have a functional TCA cycle of a higher capacity than would be deduced from their oxidation of glucose but it also highlights the effectiveness of acetoacetate as a potential energy-yielding substrate. Indeed, the combination of acetoacetate plus pyruvate proved to be the most successful in terms of calculated ATP-yield. This observation has obvious implications for the design of growth medium for the culture of hMSCs. Glucose and glutamine, the usual substrates included in standard growth medium, are not as effective in producing ATP and it could be that the design of a medium incorporating both a ketone body and pyruvate may stimulate energy-production by these cells. Since any form of stem cell therapy must involve the harvesting and *in vitro* culture of stem cells, this is something which may bear further scrutiny. The significance of pyruvate for cultured cells has been indicated by the observation that cells do not thrive in a pyruvate-free medium but succumb to oxidative stress and that stem cells *in vivo* may take up pyruvate released by other cells in the stroma. The results of the present invention support this suggestion as an effective energetic approach by these cells. The pyruvate may aid the cells' metabolism by being carboxylated to oxaloacetate thus increasing the capacity of the TCA cycle and enabling increased oxidation of acetoacetate and other oxidative substrates.

Ketone bodies represent a fuel of intermediate and prolonged starvation, persisting at low levels in the blood in the fed state (approximately 0.1mM) but rising after a fast of approximately 3-4 days in the human adult to levels that may be 70-80 times higher. Traditionally, ketone bodies have been regarded as a key fuel for neuronal tissues under these conditions. More recent reports have indicated that in general stem cells tend to reside in an environment where there are low levels of ROS (Suda et al, 2011), which may be a strategy to aid their survival or their maintenance of the undifferentiated state. The current findings show that rates of ROS generation are 4.5-fold lower, on a mole ROS per mole substrate oxidised basis, with an acetoacetate substrate than they are with a glucose substrate. The reduction in ROS produced with a ketone body substrate may partly explain why oxidation of acetoacetate represents an appropriate metabolic strategy for these cells and may indicate a preference for an acetoacetate substrate when a range of substrates are available. Since hMSCs have a high rate of proliferation and a higher rate of ATP-synthesis from acetoacetate, there is scope for speculation as to whether the ketogenic diet may have further applications in assisting with putative therapies based around stem cell transplantation, such as those for neurodegenerative disorders.

The effects of the UCP2-inhibitor, genipin, in increasing rates of ROS-production by 1.6-fold with a glucose and by 2-fold with an acetoacetate substrate, indicate the potential role for UCP2 in reducing ROS-production in hMSCs. From the results of the present invention, a potential role for UCP2 in indicating the availability of acetoacetate and enabling its mitochondrial transport and oxidation is possible and ought to be investigated. Moreover, it was found that hMSCs oxidise little endogenous pyruvate from glucose compared with their capacity for oxidising exogenous pyruvate.

Normoxic culture of hMSCs results in a general increase in flux through energy-yielding pathways between passage 2 and 5. This includes an increase of 2.2-fold in glycolytic flux and a 2-fold increase in rates of glucose-oxidation and 3.5-fold of acetoacetate. The reasons for this increased flux are not clear since they seem to be unrelated to either growth rate (which declines between passage 2 and 5) or to the onset of differentiation (shown by non-significant changes in mRNA levels for key markers over the same time-scale). This effect appears to be related to the oxygen tension since it is not reproduced by the cells cultured under hypoxic conditions, although cells cultured under normoxia with 3-HB also fail to show an increase in energy-generation. One further possibility is that there are changes in expression or activity of UCP2 over this time-scale, changing the cells' substrate- and pathway-preferences and also perhaps changing anion transport activity. Western blots show that UCP2 is present at both passage 2 and passage 5. The possibility that the presence of oxygen at a 20% tension during long-term culture may alter rates of electron transport and production of ROS should be a subject for investigation. If this phenomenon is shown to be real, it would have implications for the long-term culture of stem cells under normoxic conditions for therapeutic or research purposes since any prolonged increase in ROS-production may result in damage to DNA and higher rates of mutation during long-term culture.

The findings may also shed light on beneficial *in vivo* conditions for proliferation of stem cells in order to facilitate putative stem cell therapy.

**Table S1: Changes in mRNA-production for marker proteins at passage 5 compared with passage 2.**

| | **Fold change in mRNA** | |
|---|---|---|
| **Marker** | **Normoxic** | **Hypoxic** |
| **CD73** | 9.2 ± 0.9 | 1.000 ± 0.005 |
| **CD105** | 9.40 ± 0.89 | 0.500 ± 0.005 |
| **CD44** | 1.000 ± 0.005 | 30.0 ± 2.1 |
| **CD146** | 0.100 ± 0.005 | 0.100 ± 0.005 |
| **CD90** | 0.100 ± 0.005 | 0.100 ± 0.005 |
| **Stro1** | 0.100 ± 0.005 | 0.100 ± 0.005 |
| **Nanog** | 0.130 ± 0.004 | 1.600 ± 0.009 |
| **Oct4** | 4.9 ± 0.5 | 119 ± 8 |
| **cMyc** | 1.200 ± 0.005 | 60 ± 3 |
| **Sox2** | 0.810 ± 0.008 | 111 ± 4 |
| **TERT** | 0.044 ± 0.002 | 7.0 ± 0.4 |
| **PPARγ** | 0.46 ± 0.01 | 0.470 ± 0.003 |
| **Osteopontin** | 1.000 ± 0.005 | 239 ± 5 |
| **BMP2** | 1.000 ± 0.005 | 1.000 ± 0.005 |
| **CD45** | 0.93 ± 0.04 | 32 ± 1 |
| **CD11b** | 3.00 ± 0.05 | 1.000 ± 0.005 |
| **Notch1** | 2.60 ± 0.04 | 24 ± 2 |

Measurements of mRNA-synthesis were made by qPCR and values are expressed as the fold change at passage 5 relative to passage 2 (calculated by the 2^{-ΔΔCT} method) when hMSCs were grown under conditions of either normoxia (20% O₂) or hypoxia (5% O₂). Mesenchymal stem cell markers (CD105, CD73, CD44); general markers of pluripotency (Nanog, Oct4, cMyc, Sox2, TERT, PPARY) and of differentiated cells (Osteopontin, BMP2, CD45, CD11b, Notch1) are shown.

**Table S2: Measurements of fat, calcium and collagen for hMSCs differentiated along adipocytic, osteogenic or chondrocytic lineages.**

| A: Absorbance at 500nm for cells stained with oil red O to measure triacylglycerol and cholesteryl ester accumulation. | | | |
|---|---|---|---|
| Absorbance of eluate at 500nm/culture condition | Adipocyte-differentiated cells | Control cells | % increase over control |
| Normoxia | *** 0.556 ± 0.001 | 0.473 ± 0.003 | 18 |
| Hypoxia | *** 0.638 ± 0.001 | 0.510 ± 0.003 | 25 |
| Normoxia + 5mM 3-HB | *** 0.698 ±0.005 | 0.520 ± 0.006 | 34 |
| Hypoxia + 5mM 3-HB | *** 0.727 ± 0.001 | 0.570 ± 0.001 | 27 |

Cells were inoculated at a density of 2x10⁵ cells/well into 6 well plates and the adipocyte differentiation protocol followed (refer to Experimental Procedures for full protocol). After 18 days, medium was removed from wells and cells stained with oil red O. The dye was eluted from the cells and the absorbance of the eluate read at 500 nm.

**B: Absorbance at 577nm for cells treated with cresopthalein to measure Ca²⁺ accumulation.**

| Absorbance at 577nm/culture condition | Osteocyte-differentiated cells | Control cells | % increase over control |
|---|---|---|---|
| Normoxia | ** 0.385 ± 0.005 | 0.310 ± 0.003 | 24 |
| Hypoxia | *** 0.553 ± 0.009 | 0.376 ± 0.007 | 47 |
| Normoxia + 5mM 3-HB | *** 0.375 ± 0.003 | 0.310 ± 0.001 | 21 |
| Hypoxia + 5mM 3-HB | *** 0.495 ± 0.007 | 0.363 ± 0.003 | 36 |

Cells were inoculated at a density of 3x10⁴ cells/well into 6 well plates and the osteocyte differentiation protocol followed (refer to Experimental Procedures for full protocol). After 21 days, cells were incubated with 0.6M HCl for 24 hours before calcium per well was measured by complexing with cresopthalein and measuring absorbance at 577nm.

**C: Absorbance at 540nm for cells treated with Sirius red/fast green dye to measure collagen accumulation.**

| Absorbance at 540nm/culture condition | Chondrocyte-differentiated cells | Control cells | % increase over controls |
|---|---|---|---|
| Normoxia | ** 0.410 ± 0.020 | 0.293 ± 0.004 | 40 |
| Hypoxia | *** 0.967 ± 0.012 | 0.643 ± 0.012 | 50 |
| Normoxia + 5mM 3-HB | *** 0.887 ± 0.020 | 0.319 ± 0.004 | 178 |
| Hypoxia + 5mM 3-HB | ** 0.806 ± 0.024 | 0.556 ± 0.008 | 45 |

Cells were inoculated at a density of 2x10⁵ cells/well into 6 well plates and the chondrocyte-differentiation protocol followed (refer to Experimental Procedures for full protocol). After 21 days, cells were fixed with Kahle fixative and stained with Sirius dye. Dye was extracted and the absorbance read at 540nm.
n=3
** p<0.004; *** p<0.0001

### Non-patent references:

Schofield, R. (1978) The relationship between the spleen colony-forming cell and the haemopoietic stem cell Blood Cells 4 7-25.
Crisan M, Yap S, Casteilla L, Chen CW, Corselli M, Park TS. (2008) A perivascular origin for mesenchymal stem cells in multiple human organs. Cell Stem Cell. 3 301-313.
Parmar K, Mauch P, Vergilio JA, Sackstein R, Down JD. (2007) Distribution of hematopoietic stem cells in the bone marrow according to regional hypoxia. Proc Natl Acad Sci U S A. 104 5431-5436.
Suda, T., Takubo, K. and Semenza, G.L. (2011) Metabolic Regulation of Hematopoietic Stem Cells in the Hypoxic Niche Cell Stem Cell 9 (4) 298-310.
Pattappa, G., Heywood, H.K., De Bruijn, J.D., and Lee, D.A. (2011) The Metabolism of Human Mesenchymal Stem Cells During Proliferation and Differentiation J. Cell. Physiol. 226 2562-70.
Simsek, T., Kocobas, F., Zheng, J., DeBarardinis, R.J., Mahmoud, A.I., Olson, E.N., Schneider, J.W., Zhang, C.C. and Sadek, H.A. (2010) The Distinct Metabolic Profile of Hematopoietic Stem Cells Reflects Their Location in a Hypoxic Niche Cell Stem Cell 7 (3) 380-90.
Gaspar, J.A., Doss, M.X., Hengstler, J.G., Cadenas, C., Hescheler, J. and Sachinidis, A. (2014) Unique Metabolic Features of Stem Cells, Cardiomyocytes, and Their Progenitors Circ. Res. 114 1346-60.
Collins, C.L., Bode, B.P., Souba, W.W. and Abcouwer, S.F. (1998) Multiwell 14CO2-capture assay for evaluation of substrate oxidation rates of cells in culture. Biotechniques, 24 (5) 803-8.
Hammerstedt, R.H. (1973) The use of Dowex-1-borate to separate 3H2O from 2-3H-glucose Anal. Biochem. 56 292-3.
Rada,T., Reis, R.L. and Gomes, M.E. (2011) Distinct stem cells subpopulations isolated from human adipose tissue exhibit different chondrogenic and osteogenic differentiation potential Stem Cell Rev. 7(1) 64-76.
Furstenberger, G., von Moos, R., Senn, H.J. and Boneberg, E.M. (2005) Real-time PCR of CD146 mRNA in peripheral blood enables the relative quantification of circulating endothelial cells and is an indicator of angiogenesis Br. J. Cancer 93 (7) 793-8.
Li, J., Xin, J., Zhang, L., Wu, J., Jiang, L., Zhou, Q., Li, J., Guo, J., Cao, H. and Li, L. (2013) Human hepatic progenitor cells express hematopoietic cell markers CD45 and CD109 Int. J. Med. Sci. 11(1) 65-79.
Wilson, K.D., Venkatasubrahmanyam, S., Fu, J.D., Sun, N., Abilez, O.J., Baugh, J.J., Jia, F., Ghosh, Z., Li, R.A., Butte, A.J. and Wu, J.C. (2010) Dynamic microRNA expression programs during cardiac differentiation of human embryonic stem cells: role for miR-499 Circ. Cardiovasc. Genet. 3(5) 426-35.
Liu, L., Wei, X., Ling, J., Wu, L. and Xiao, Y. (2011) Expression pattern of Oct-4, Sox2, and c-Myc in the primary culture of human dental pulp derived cells. J. Endod 37 (4) 466-72.
Friedrich, M.G., Weisenberger DJ, Cheng JC, Chandrasoma S, Siegmund KD, Gonzalgo ML, Toma MI, Huland H, Yoo C, Tsai YC, Nichols PW, Bochner BH, Jones PA, Liang G. (2004) Detection of methylated apoptosis-associated genes in urine sediments of bladder cancer patients Clin Cancer Res. 10(22):7457-65.
Zhang Y, Ba Y, Liu C, Sun G, Ding L, Gao S, Hao J, Yu Z, Zhang J, Zen K, Tong Z, Xiang Y and Zhang CY (2007) PGC-1alpha induces apoptosis in human epithelial ovarian cancer cells through a PPARgamma-dependent pathway Cell Res. 17(4) 363-73.
Pera MF, Andrade J, Houssami S, Reubinoff B, Trounson A, Stanley EG, Wardvan Oostwaard D, Mummery C. (2004) Regulation of human embryonic stem cell differentiation by BMP-2 and its antagonist noggin J Cell Sci. 117(Pt 7) 1269-80.
Zhou X, Gao XP, Fan J, Liu Q, Anwar KN, Frey RS and Malik AB. (2005) LPS activation of Toll-like receptor 4 signals CD11b/CD18 expression in neutrophils Am J Physiol Lung Cell Mol Physiol. 288(4) L655-62.
Pilz GA, Braun J, Ulrich C, Felka T, Warstat K, Ruh M, Schewe B, Abele H, Larbi A and Aicher WK. (2011) Human mesenchymal stromal cells express CD14 cross-reactive epitopes Cytometry A. 79(8):635-45.
Okumoto K, Saito T, Hattori E, Ito JI, Adachi T, Takeda T, Sugahara K, Watanabe H, Saito K, Togashi H and Kawata S. (2003) Differentiation of bone marrow cells into cells that express liver-specific genes in vitro: implication of the Notch signals in differentiation Biochem Biophys Res Commun.304(4) 691-5.
Livak, K.J. and. Schmittgen, T.D. (2001) Analysis of Relative Gene Expression Data Using Real-Time Quantitative PCR and the 2-ΔΔCT Method Methods 25, 402-408.
Heather, L.C., Howell, N.J., Emmanuel, Y., Cole, M.A., Frenneaux, M.P., Pagano, D. and Clarke, K. (2011) Changes in cardiac substrate transporters and metabolic proteins mirror the metabolic shift in patients with aortic stenosis PLOS one 6 (10) e26326 doi 10.1371/journal.pone.0026326.
Jager, M., Feser, T., Denck, H. and Kraupse, R. (2005) Proliferation and osteogenic differentiation of mesenchymal stem cells cultures onto three different polymers in vitro Annals Biomed. Engineering 33 (10) 1319-32. Negre-Salvayre, A., Hirtz, C., Carrera, R., Cazenave, R., Troly, M., Salvayre, L., Penicaud, L. and Casteilla, L. (1997) A role for uncoupling protein-2 as a regulator of mitochondrial hydrogen peroxide generation FASEB J. 11 809-15.
Arsenijevic, D., Onuma, H., Percqueur, C., Raimbault, S., Manning, B.S., Miroux, B., Couplan, E., Alves-Guerra, M.C., Goubern, M., Surwit, R., Bouilaud, F., Richard, D., Collins, S. and Ricquier, D. (2000) Disruption of the uncoupling protein-2 gene in mice reveals a role in immunity and reactive oxygen species production Nat. Genet. 26 435-9.
Zhang,C.Y.,. Parton,L.E. Ye,C.P Krauss,S. Shen,R. Lin,C-T, Porco J.A. and Lowell B.B. (2006) Genipin inhibits UCP2-mediated proton leak and acutely reverses obesity- and high glucose-induced β cell dysfunction in isolated pancreatic islets Cell Metabolism 3 417-427.
Ledesma, A., Garcia de Lacoba, M. and Rial, E. (2002) The mitochondrial uncoupling proteins Genme Biol. 3 reviews 3015.9 doi : 10.1186/gb-2002-3-12-reviews 3015.
Trézéguet,V., Le Saux, A., David, C., Gourdet, C., Fiore, C., Dianoux, A., Brandolin, G. and Lauquin, G.J. (2000) A covalent tandem dimer of the mitochondrial ADP/ATP carrier is functional in vivo Biochim. Biophys. Acta 1457 (1-2), 81-93.

## Claims

1. Method for cultivating primary Mesenchymal Stem Cells (MSCs) *in vitro,* wherein the cells are cultivated in a culture medium comprising at least one ketone body.

2. The method according to claim 1, wherein the cells are cultivated in a gas atmosphere comprising an oxygen concentration of about 10 - 30 % by volume, preferably 15 - 25 % or 19 - 22 % by volume, in particular 20 - 21 % by volume.

3. The method according to claim 1 or 2, wherein the ketone body is selected from the group consisting of acetoacetate (3-oxobutyrate), *beta-*hydroxybutyrate (3- hydroxybutyrate), *beta*-ketopentanoate (3-oxopentanoate), and *beta*-hydroxypentanoate (3- hydroxypentanoate).

4. The method according to any one of claims 1 to 3, wherein the culture medium further comprises pyruvate.

5. The method according to any one of claims 1 to 4, wherein the culture medium is devoid of glucose or any precursor thereof.

6. The method according to any one of claims 1 to 5, wherein said Mesenchymal Stem Cells (MSCs) are human Mesenchymal Stem Cells (hMSCs).

7. Use of a culture medium comprising at least one ketone body for cultivating primary Mesenchymal Stem Cells (MSCs) *in vitro.*

8. The use according to claim 7, wherein the ketone body is selected from the group consisting of acetoacetate (3-oxobutyrate), *beta*-hydroxybutyrate (3-hydroxybutyrate), *beta*-ketopentanoate (3-oxopentanoate), and *beta-*hydroxypentanoate (3- hydroxypentanoate).

9. The use according to claim 7 or 8, wherein said culture medium further comprises pyruvate.

10. The use according to any one of claims 7 to 9, said culture medium being devoid of glucose or any precursor thereof.

11. The use according to any one of claims 7 to 10, wherein said culture medium further comprises a base medium and, optionally, at least one supplement.

## Patentansprüche

1. Verfahren zur Kultivierung von primären mesenchymalen Stammzellen (MSCs) *in vitro,* wobei die Zellen in einem Kulturmedium kultiviert werden, das mindestens einen Ketonkörper umfasst.

2. Verfahren nach Anspruch 1, wobei die Zellen in einer Gas-Atmosphäre mit einer Sauerstoffkonzentration von ungefähr 10 - 30 Vol.-%, vorzugsweise 15 - 25 Vol.-% oder 19 - 22 Vol.-%, insbesondere 20 - 21 Vol.-%, kultiviert werden.

3. Verfahren nach Anspruch 1 oder 2, wobei der Ketonkörper aus der Gruppe bestehend aus Acetoacetat (3-Oxobutyrat), beta-Hydroxybutyrat (3-Hydroxybutyrat), *beta*-Ketopentanoat (3-Oxopentanoat) und *beta-*Hydroxypentanoat (3-Hydroxypentanoat) ausgewählt ist.

4. Verfahren nach einem der Ansprüche 1 bis 3, wobei das Kulturmedium ferner Pyruvat umfasst.

5. Verfahren nach einem der Ansprüche 1 bis 4, wobei das Kulturmedium frei von Glucose oder einem von dessen Vorläufern ist.

6. Verfahren nach einem der Ansprüche 1 bis 5, wobei diese mesenchymalen Stammzellen (MSCs) humane mesenchymale Stammzellen (hMSCs) sind.

7. Verwendung eines Kulturmediums, das mindestens einen Ketonkörper umfasst, zur Kultivierung primärer mesenchymaler Stammzellen (MSCs) *in vitro.*

8. Verwendung nach Anspruch 7, wobei der Ketonkörper aus der Gruppe bestehend aus Acetoacetat (3-Oxobutyrat), *beta*-Hydroxybutyrat (3-Hydroxybutyrat), *beta*-Ketopentanoat (3-Oxopentanoat) und *beta-*Hydroxypentanoat (3-Hydroxypentanoat) ausgewählt ist.

9. Verwendung nach Anspruch 7 oder 8, wobei dieses Kulturmedium ferner Pyruvat umfasst.

10. Verwendung nach einem der Ansprüche 7 bis 9, wobei dieses Kulturmedium frei von Glucose oder einem von dessen Vorläufern ist.

11. Verwendung nach einem der Ansprüche 7 bis 10, wobei dieses Kulturmedium ferner ein Basismedium und, optional, mindestens einen Zusatz umfasst.

## Revendications

1. Procédé destiné à mettre en culture des cellules souches mésenchymateuses (CSM) *in vitro,* où les cellules sont mises en culture dans un milieu de culture comprenant au moins un corps cétonique.

2. Procédé selon la revendication 1, où les cellules sont mises en culture dans une atmosphère gazeuse comprenant une concentration en oxygène d'environ 10 à 30 % par volume, de préférence de 15 à 25 % ou de 19 à 22 % par volume, notamment 20 à 21 % par volume.

3. Procédé selon les revendications 1 ou 2, où le corps cétonique est sélectionné dans le groupe comprenant l'acétoacétate (3-oxobutyrate), le *β-*hydroxybutyrate (3-hydroxybutyrate), le *β*-cétopentanoate (3-oxopentanoate) et le *β*-hydroxypentanoate (3-hydroxypentanoate).

4. Procédé selon l'une quelconque des revendications 1 à 3, où le milieu de culture comprend en outre du pyruvate.

5. Procédé selon l'une quelconque des revendications 1 à 4, où le milieu de culture est dépourvu de glucose ou d'un quelconque précurseur de celui-ci.

6. Procédé selon l'une quelconque des revendications 1 à 5, où lesdites cellules souches mésenchymateuses (CSM) sont des cellules souches mésenchymateuses humaines (CSMh).

7. Utilisation d'un milieu de culture comprenant au moins un corps cétonique pour la mise en culture de cellules souches mésenchymateuses (CSM) primaires *in vitro.*

8. Utilisation selon la revendication 7, où le corps cétonique est sélectionné dans le groupe comprenant l'acétoacétate (3-oxobutyrate), le *β-*hydroxybutyrate (3-hydroxybutyrate), le *β*-cétopentanoate (3-oxopentanoate) et le *β*-hydroxypentanoate (3-hydroxypentanoate).

9. Utilisation selon les revendications 7 ou 8, où ledit milieu de culture comprend en outre du pyruvate.

10. Utilisation selon l'une quelconque des revendications 7 à 9, où ledit milieu de culture est dépourvu de glucose ou d'un quelconque précurseur de celui-ci.

11. Utilisation selon l'une quelconque des revendications 7 à 10, où ledit milieu de culture comprend en outre un milieu de base et, facultativement, au moins un supplément.
